# EUROPÄISCHE PATENTANMELDUNG

(11) **EP 4 588 558 A1**
(43) Veröffentlichungstag der Anmeldung: **23.07.2025**
(21) Anmeldenummer: 24152110.3
(22) Anmeldetag: 16.01.2024
(51) Int. Cl.: B01J 8/02, C07C 67/303, C07C 69/75

(54) **TEMPERATURREGELUNG IN DER HERSTELLUNG VON ALICYCLISCHEN POLYCARBONSÄUREN UND DEREN ESTERN**

(71) Anmelder: Evonik Oxeno GmbH & Co. KG, 45772 Marl (DE)
(72) Erfinder: ZIOMEK, Grzegorz, 45665 Recklingshausen (DE); SCHNEIDER, Thomas, 46514 Schermbeck (DE); GRASS, Michael, 45721 Haltern am See (DE); SIMON, Berno, 45772 Marl (DE)
(74) Vertreter: Evonik Patent Association

(57) **Zusammenfassung**

Die Erfindung liegt auf dem technischen Gebiet der Herstellung von alicyclischen Verbindungen durch Kernhydrierung von aromatischen Verbindungen. Es werden im Rahmen der Erfindung Verfahren zur Herstellung alicyclischer Verbindungen, vorzugsweise alicyclischer Carbonsäuren und deren Estern, bereitgestellt sowie apparative Vorrichtungen zur Durchführung dieses Verfahrens.

## Beschreibung

Die Erfindung liegt auf dem technischen Gebiet der Herstellung von alicyclischen Verbindungen durch Kernhydrierung von aromatischen Verbindungen. Es werden im Rahmen der Erfindung Verfahren zur Herstellung alicyclischer Verbindungen, vorzugsweise alicyclischer Carbonsäuren und deren Estern, bereitgestellt sowie apparative Vorrichtungen zur Durchführung dieses Verfahrens.

Alicyclische Polycarbonsäureester, wie beispielsweise die Ester der Cyclohexan-1,2-dicarbonsäure, werden als Schmierölkomponente und als Hilfsmittel bei der Metallverarbeitung eingesetzt. Weiterhin finden sie als Weichmacher für Polyolefine und für PVC Verwendung.

Für die Weichmachung von PVC werden überwiegend Ester der Phthalsäure, wie beispielweise Dinonyl- oder Didecylester, verwendet. Die Verwendung dieser Phthalate wird in der Öffentlichkeit zunehmend kontrovers diskutiert und die Verwendung in Kunststoffen könnte limitiert werden. Alicyclische Polycarbonsäureester, von denen einige in der Literatur bereits als Weichmacher für Kunststoffe beschrieben sind, können eine geeignete Wahl für mögliche Ersatzstoffe der limitierten Weichmacher darstellen.

In den meisten Fällen ist der wirtschaftlichste Weg zur Herstellung von alicyclischen Polycarbonsäureestern die Kernhydrierung der entsprechenden aromatischen Polycarbonsäureester, beispielsweise der o. g. Phthalate. Es sind hierzu bereits einige Verfahren bekannt:
In DE 102 32 868.4 und DE 102 25 565.2 wird die Hydrierung der aromatischen Polycarbonsäureester zu den entsprechenden alicyclischen Polycarbonsäureestern in zwei hintereinandergeschalteten Reaktoren durchgeführt, wobei der erste in Schlaufenfahrweise (teilweise Rückführung des Reaktoraustrages) und der zweite im geraden Durchgang betrieben wird. Der erste Schlaufenreaktor kann auch durch mehrere kleine in Reihe oder parallel verschaltete Schlaufenreaktoren ersetzt werden, wobei diese Reaktoren einen gemeinsamen Kreislauf aufweisen.

Die Anmeldung EP 1 676 829 offenbart ein Verfahren zur kontinuierlichen katalytischen Hydrierung in mindestens zwei hintereinander geschalteten Reaktoren, wobei das Katalysatorvolumen möglichst geringgehalten wird. Hierbei wird der erste Reaktor in Schlaufenfahrweise betrieben und mindestens ein weiterer im geraden Durchgang. Da die Hydrierung ein exothermes Verfahren ist, wird zu Beginn eines solchen Verfahrens mit frischem Katalysator eine recht hohe Temperatur im ersten Reaktor, welcher in Schlaufenfahrweise betrieben wird, erreicht. Über die Zeit der kontinuierlichen Hydrierung sinkt die Aktivität des Katalysators ab. Das Absinken der Aktivität des Katalysators im ersten Reaktor wird dann üblicherweise dadurch abgefangen, dass die Temperatur im ersten Reaktor erhöht wird. Die Erhöhung der Temperatur im ersten Reaktor kann jedoch zu Problemen im zweiten Reaktor, der im gerade Durchgang betrieben wird, führen. Aufgrund sicherheitsrelevanter Aspekte kann die Temperatur im ersten und im zweiten Reaktor nicht unendlich hoch werden. Dies kann dazu führen, dass der erste Reaktor nicht mehr bei ausreichend hoher Temperatur gefahren werden kann, da sonst eine temperaturbedingte Abschaltung des ersten und insbesondere des zweiten Reaktors aufgrund von Sicherheitsaspekten drohen kann. Eine frühzeitige Abschaltung des Reaktors kann zu einer verringerten Ausbeute und zu einer unzureichenden Hydrierung des Edukts im Produktstrom des zweiten Reaktors führen.

Die primäre Aufgabe der vorliegenden Erfindung war es daher, ein Verfahren zur katalytischen Hydrierung von aromatischen Verbindungen, vorzugsweise von aromatischen Polycarbonsäuren und deren Estern, bereitzustellen, welches eine hohe Ausbeute besitzt und bei welchem der Produktstrom einen gleichbleibend hohen Anteil von alicyclischen Verbindungen als Produkt der Hydrierung der eingesetzten aromatischen Verbindungen enthält.

Diese primäre Aufgabe wurde dadurch gelöst, dass ein Verfahren zur Herstellung von einer oder mehrerer alicyclischer Verbindungen, bereitgestellt wird, umfassend die Schritte:
i. Bereitstellen eines Stroms A (2b), umfassend eine oder mehrere aromatische Verbindungen und eines wasserstoffhaltigen Hydriergases;
ii. Zuführen des Stroms A (2b) mit einer Temperatur T₁ zu einer ersten Hydriereinheit (1) und Hydrieren der einen oder mehreren aromatischen Verbindungen zu einer oder mehrerer entsprechender alicyclischer Verbindungen;
iii. Erhalten eines Gemisches als ersten Produktstrom (3) mit einer Temperatur T₂ umfassend aromatische Verbindungen und alicyclische Verbindungen;
iv. Auftrennen des in Schritt iii. erhaltenen Produktstroms (3) in einen Teilstrom (8), der zu dem Strom A (2b) in Schritt i. zugeführt und als Teil des Stroms A (2b) in Schritt ii. erneut hydriert wird, sowie einen Teilstrom (6c), der als Strom B (9) bei einer Temperatur T₃ in eine oder mehrere weitere Hydriereinheiten (11) zugeführt wird;
v. Hydrieren der in Strom B (9) enthaltenen aromatischen Verbindungen zu den entsprechenden alicyclischen Verbindungen in der einen oder mehreren weiteren Hydriereinheiten (11), und
vi. Erhalten eines Produktstroms (12) mit einer Temperatur T₄ umfassend eine oder mehrere alicyclische Verbindungen entsprechend zu der in Schritt i. bereitgestellten einen oder mehreren aromatischen Verbindungen,
wobei zumindest zeitweise die Temperatur T₁ ungleich der Temperatur T₃ ist, vorzugsweise wobei die Temperatur T₁ höher ist als die Temperatur T₃.

Unter "alicyclischen Verbindungen" im Rahmen der vorliegenden Erfindung sind solche Verbindungen zu verstehen, die ein gesättigtes Ringsystem mit einer aliphatischen Struktur besitzen. Solche Verbindung sind auch als cycloaliphatische Verbindungen bekannt. Vorzugsweise besitzen die alicyclischen Verbindungen, die im Rahmen der vorliegenden Erfindung als Produkte erhalten werden, einen Cylohexanring.

Unter "aromatischen Verbindungen" im Rahmen der vorliegenden Erfindung sind solche Verbindungen zu verstehen, die mindestens ein Ringsystem besitzen, das nach der Hückel-Regel in konjugierten Doppelbindungen, freien Elektronenpaaren oder unbesetzten p-Orbitalen eine Anzahl von 4n+2 delokalisierten Elektronen enthält. Vorzugsweise besitzen die aromatischen Verbindungen, die im Rahmen der vorliegenden Erfindung als Edukte eingesetzt werden, einen Benzolring.

Ein "wasserstoffhaltiges Hydriergas" ist ein Gas, welches Wasserstoff enthält. Wasserstoff wird im Rahmen der der vorliegenden Erfindung zu Grunde liegenden Reaktion neben den aromatischen Verbindungen als weiteres Edukt eingesetzt. Bei der durchgeführten Hydrierreaktion werden die Doppelbindungen im Ring der eingesetzten aromatischen Verbindungen, vorzugsweise des Benzolrings, durch eine Additionsreaktion des Wasserstoffs hydriert und dadurch aufgelöst. Die Reaktion findet in Anwesenheit eines Feststoffkatalysators statt. Hierbei bindet das Wasserstoffmolekül auf dem wasserstoffhaltigen Hydriergas intermediär an das Metallatom des Katalysators und die Bindung zwischen den beiden Wasserstoffatomen im Wasserstoffmolekül wird geschwächt und kann mit einer elektronenreichen Mehrfachbindung (Doppelbindung) wechselwirken. Die Hydrierung findet statt, wenn formal jeweils zwei Wasserstoffatome auf eine Doppelbindung übertragen werden. Damit werden die Doppelbindungen in den aromatischen Verbindungen aufgelöst und es werden alicyclische Verbindungen erhalten.

Als Hydriergase können beliebige wasserstoffhaltige Gasgemische, die keine schädlichen Mengen an Katalysatorgiften, wie beispielsweise Kohlenmonoxid oder Schwefelwasserstoff enthalten, eingesetzt werden. Die Verwendung von Inertgasen ist optional, bevorzugt wird Wasserstoff in einer Reinheit größer 95 %, insbesondere größer 98 % eingesetzt. Lnertgasanteile können beispielsweise Stickstoff oder Methan sein. Vorzugsweise ist in den Hydriereinheiten so viel Wasserstoff vorhanden, dass dieser im Überschuss, insbesondere in einem Überschuss von 1 bis 200 %, bevorzugt in einem Überschuss von 3 bis 100 % und besonders bevorzugt in einem Überschuss von 5 bis 50 % bezogen auf die stöchiometrische Menge, die zur Erzielung des in der Hydriereinheit möglichen bzw. gewünschten Umsatzes benötigt wird, vorliegt. Das Einstellen eines ausreichenden Überschusses an Wasserstoff kann sich vorteilhaft auf die vollständige Hydrierung der aromatischen Bindungen auswirken.

Eine "Hydriereinheit" im Rahmen der vorliegenden Erfindung ist zu verstehen als Hydrierreaktor oder mehrere hintereinandergeschalteter Reaktoren oder mehrere parallel zueinander geschalteten Reaktoren oder eine Reaktorgruppe, die aus parallel und hintereinander geschalteten Reaktoren besteht. Daher zu verstehen als ein Reaktor oder eine Reaktoranordnung, die im erfindungsgemäßen Verfahren die Funktion eines Reaktors ausüben kann.

Die einzelnen Hydriereinheiten können mit Frisch-Wasserstoff beschickt werden. Um den Wasserstoffverbrauch und die mit dem Abgas bedingten Austragsverluste zu minimieren, ist es jedoch zweckmäßig, das Abgas einer Hydriereinheit als Hydriergas einer anderen oder der gleichen Hydriereinheit zu verwenden. Weiterhin kann das Abgas einer Hydriereinheit nach Aufarbeitung wieder als Frisch-Wasserstoff eingesetzt werden. Beispielsweise ist es bei einem Verfahren, das in zwei hintereinander geschalteten Hydriereinheiten mit jeweils einer Hydriereinheit durchgeführt wird, vorteilhaft, Frisch-Wasserstoff in die erste Hydriereinheit einzuspeisen und das Abgas der ersten Hydriereinheit in die zweite Hydriereinheit zu leiten. In diesem Falle strömen Edukt und Hydriergas in entgegengesetzter Reihenfolge durch die Hydriereinheiten. Es ist bei dieser Verfahrensführung vorteilhaft, den Wasserstoffüberschuss, bezogen auf die stöchiometrisch notwendige Menge, unter 30 %, insbesondere unter 20 % zu halten.

Unter "Rezyklieren" oder "Schlaufenfahrweise" versteht man im Rahmen der vorliegenden Erfindung das zumindest teilweise Rückführen des Produktstroms einer Hydriereinheit als Teil des Eingangsstroms zu derselben Hydriereinheit. Der Produktstrom bzw. das aus einer Hydriereinheit erhaltene Gemisch wird dabei aufgeteilt. Das heißt, dass ein Teilstrom des Hydrieraustrags bzw. Produktstroms der ersten Hydriereinheit zusammen mit Frisch-Edukt als Strom A in die erste Hydriereinheit geleitet wird. Der andere Teilstrom des Hydrieraustrags bzw. Produktstroms der ersten Hydriereinheit wird in einer zweiten Hydriereinheit, die vorzugsweise im geraden Durchgang betrieben wird, hydriert. Es ist auch möglich, anstelle einer großen Hydriereinheit in Schlaufenfahrweise mehrere kleinere Einheiten, die in Reihe oder parallel angeordnet sind, zu verwenden. Ebenso ist es möglich, anstatt einer großen Hydriereinheit, die im geraden Durchgang durchströmt wird, mehrere Einheiten, die in Reihe oder parallel miteinander verschaltet sind, zu betreiben. Bevorzugt wird jedoch nur eine Hydriereinheit, die in Schlaufenfahrweise betrieben wird und eine Einheit, die im geraden Durchgang betrieben wird, verwendet. Das Verfahren kann auch in Rohrbündelreaktoren durchgeführt werden.

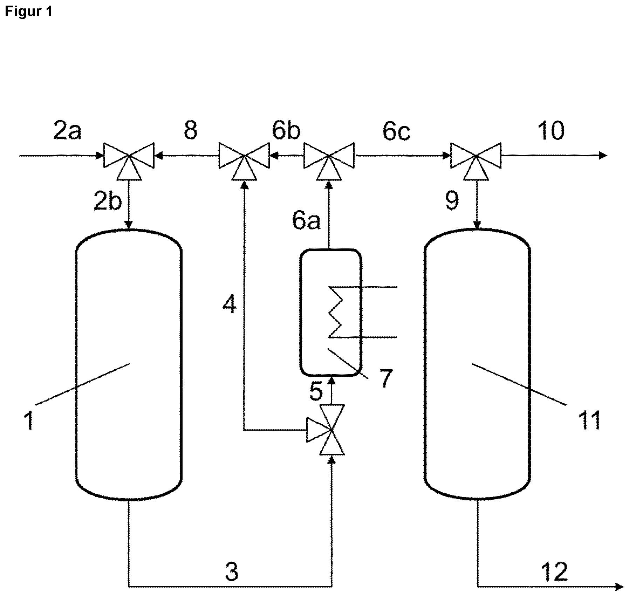

Am Beispiel der Figur 1 bedeutet dies, dass der Produktstrom (3) über die Rohrleitungen (4,8) zusammen mit Frisch-Edukt (2a) als Eingangsstrom beziehungsweise Strom A (2b) zu der Hydriereinheit (1) zugeführt werden kann.

Bevorzugt im Rahmen des erfindungsgemäßen Verfahrens ist es, wenn Schritt iv. mit einem Kreislaufverhältnis von 1 zu 10 bis 1 zu 50, vorzugsweise von 1 zu 10 bis 1 zu 40, besonders bevorzugt von 1 zu 30 durchgeführt wird. Dieses Verhältnis bedeutet am Wert 1 zu 10, dass beispielsweise zehn Tonnen des Produktstroms wieder auf den Kopf der ersten Hydriereinheit zugeführt werden und eine Tonne der mindestens einen weiteren Hydriereinheit zugeführt wird.

Das Kreislaufverhältnis wird vorzugsweise so eingestellt, dass in der ersten der hintereinandergeschalteten Hydriereinheiten ein Gesamtumsatz von 80 bis 99 %, bevorzugt von 85 bis 97 % und in der zweiten Hydriereinheit ein Umsatz von 80 bis 100 %, bevorzugt von 85 bis 100 % bezogen auf die Ausgangskonzentration der zu hydrierenden Verbindung am Eingang der jeweiligen Hydriereinheit erzielt wird. Falls drei oder mehr Hydriereinheiten verwendet werden, müssen die Umsätze entsprechend angepasst werden.

Die mindestens eine weitere Hydriereinheit kann ebenfalls in Schlaufenfahrweise oder im geraden Durchgang betrieben werden, was bedeutet, dass keine Rückführung des Rezyklats in die gleiche Hydriereinheit stattfindet.

Die Hydrierung kann in Ab- oder vorzugsweise in Anwesenheit eines Lösungsmittels durchgeführt werden. Als Lösemittel können alle Flüssigkeiten eingesetzt werden, die mit dem Edukt und Produkt eine homogene Lösung bilden, sich unter Hydrierbedingungen inert verhalten und sich leicht vom Produkt abtrennen lassen. Das Lösemittel kann auch ein Gemisch mehrerer Stoffe sein und gegebenenfalls Wasser enthalten.

Beispielsweise können folgende Stoffe als Lösemittel eingesetzt werden: Geradkettige oder cyclische Ether, wie beispielsweise Tetrahydrofuran oder Dioxan, sowie aliphatische Alkohole, in denen der Alkylrest 1 bis 13 Kohlenstoffatome aufweist.

Bevorzugt verwendbare Alkohole sind Isopropanol, n-Butanol, Isobutanol, n-Pentanol, 2-Ethylhexanol, Nonanole, technische Nonanolgemische, Decanol, technische Decanolgemische, Tridecanole.

Bei Einsatz von Alkoholen als Lösemittel kann es zweckmäßig sein, denjenigen Alkohol oder dasjenige Alkoholgemisch zu verwenden, das bei der Verseifung des Produkts entstehen würde. Dadurch würde die Nebenproduktbildung durch Umesterung ausgeschlossen. Ein weiteres bevorzugtes Lösemittel ist das Hydrierprodukt selbst.

Durch die Verwendung eines Lösemittels kann die Konzentration an aromatischen Verbindungen im Reaktorzulauf begrenzt werden, wodurch eine bessere Temperaturkontrolle im Reaktor erreicht werden kann. Dies kann eine Minimierung von Nebenreaktionen und somit eine Erhöhung der Produktausbeute zur Folge haben. Bevorzugt liegt kann die Konzentration an aromatischen Verbindungen im Reaktorzulauf zwischen 1 und 35 Gew.-%, insbesondere zwischen 5 und 25 Gew.-% bezogen auf die Gesamtmenge des eingesetzten Edukts. Der gewünschte Konzentrationsbereich kann bei Reaktoren, die in Schlaufenfahrweise betrieben werden, durch das Kreislaufverhältnis (Mengenverhältnis von rückgeführten Hydrieraustrag zu Edukt) eingestellt werden.

Nachfolgend wird das erfindungsgemäße Verfahren am Beispiel des in Figur 1 gezeigten Aufbaus beispielsweise beschrieben:
In Schritt i. werden als Edukte mindestens eine aromatische Verbindung, sowie ein wasserstoffhaltiges Hydriergas bereitgestellt und über den Eingangsstrom als Strom A (2b) in eine erste Hydriereinheit (1) zugeleitet. Hierbei wird im Eingangsstrom (2a) Frisch-Edukt zugeführt und über den Eingangsstrom (2b) der ersten Hydriereinheit zugeleitet. Der Eingangsstrom (2b) besitzt die Temperatur T₁. In dieser ersten Hydriereinheit (1) findet dann die Hydrierung in Schritt ii. statt und es wird in Schritt iii. am Ende der Hydriereinheit ein Gemisch als Produktstrom (3) mit hydrierten Verbindungen (alicyclischen Verbindungen) und nicht hydrierten Verbindungen (aromatischen Verbindungen) erhalten. Dieses Gemisch bzw. der Produktstrom (3) besitzt die Temperatur T₂ und wird anschließend in Schritt iv. ein zwei Teilströme aufgeteilt, wobei der eine Teilstrom als Strom (8) dem Strom A (2b) zugeleitet wird und mit Frisch-Edukt (2a) in der ersten Hydriereinheit (1) einer erneuten Hydrierung gemäß Schritt ii. unterzogen wird. Der zweite Teilstrom Strom B (6c) wird über den Eingangsstrom (9) in eine zweite Hydriereinheit (11) eingeleitet und besitzt die Temperatur T₃. Dieser wird in mindestens einer weiteren Hydriereinheit (11) in Schritt v. hydriert, sodass das Edukt, was in der ersten Hydriereinheit (1) nicht umgesetzt wurde, in dieser zweiten Hydriereinheit (11) zu den entsprechenden alicyclischen Verbindungen hydriert wird. Das in Schritt vi. als Strom (12) erhaltene Produktgemisch besitzt die Temperatur T₄. Die Aufteilung der Produktströme in Schritt iv. kann entweder über einen Bypass als Strom (4) erfolgen oder über eine Kühleinrichtung (7), vorzugsweise über einen Wärmetauscher.

Der Produktstrom (12) am Ausgang der mindestens einen weiteren Hydriereinheit enthält vorzugsweise weniger als 0,3 Massen-%, vorzugsweise weniger als 0,1 Massen-%, insbesondere weniger als 0,05 Massen-%, besonders bevorzugt 0,005 Massen-% als Edukt eingesetzter alicyclischer Verbindungen.

Vorzugsweise werden die Prozessparameter, wie Produkt-, Nebenprodukt- und Eduktkonzentration sowie Temperatur mittels Online-Analytik bestimmt. Die Online-Analytik erfasst die jeweiligen Parameter in Echtzeit, vorzugsweise in den Produktausgangsströmen der ersten (3) und/oder jederweiteren Hydriereinheit (12). Vorzugsweise wird eine Messmethode ausgewählt aus der Gruppe bestehend aus Reaktionskalorimeter, ATR-FT-IR Spektroskopie, RAMAN-Spektroskopie, IR-Spektroskopie, UV-und/oder UV-VIS Spektroskopie oder Kombinationen davon, verwendet. Anhand der damit bestimmten Prozessparameter ließe sich nach vorheriger Bestimmung eines Grenzwerts die Temperatur T₁ gezielt einstellen. Diese Einstellung kann auch automatisiert, also unter Zuhilfenahme von Computertechnik, erfolgen.

Es ist bevorzugt im Zusammenhang mit der vorliegenden Erfindung, dass das Hydrieren der in Schritt i. bereitgestellten aromatischen Verbindungen an einem oder mehreren in einem Festbett der Hydriereinheiten angeordneten Feststoff-Katalysatoren mit dem in Schritt i. bereitgestellten wasserstoffhaltigen Gas erfolgt.

Es ist weiterhin bevorzugt, dass der Feststoff-Katalysator mindestens ein Metall der achten Nebengruppe des Periodensystems der Elemente aufweist. Vorzugsweise werden als Aktivmetalle Platin, Rhodium, Palladium, Kobalt, Nickel oder Ruthenium oder ein Gemisch aus zwei oder mehreren davon eingesetzt, wobei insbesondere Ruthenium als Aktivmetall eingesetzt wird.

Neben den bereits genannten Metallen kann zusätzlich mindestens ein Metall der ersten und/oder siebten Nebengruppe des Periodensystems der Elemente in den Katalysatoren enthalten sein. Bevorzugt wird Rhenium und/oder Kupfer zusätzlich zu dem Metall der achten Nebengruppe des Periodensystems der Elemente eingesetzt.

Die im Rahmen dieses Verfahren eingesetzte Katalysatoren sind vorzugsweise auf ein Trägermaterial aufgebrachte Metalle wie vorhergehend definiert. Vorzugsweise handelt es sich bei den eingesetzten Trägermaterialien um Materialien enthaltend Mikroporen (Porendurchmesser kleiner 2 nm), Mesoporen (Porendurchmesser 2 bis 50 nm) und Makroporen (Porendurchmesser größer 50 nm) unterschieden werden. So sind hinsichtlich der Porenart Trägermaterialien mit folgenden Porenkombinationen einsetzbar:
a) nur Mesoporen,
b) Mikroporen und Mesoporen,
c) Mesoporen und Makroporen,
d) Mikroporen und Mesoporen und Makroporen,
e) Mikroporen und Makroporen.

Vorzugsweise werden als Trägermaterialien Aktivkohle, Siliciumcarbid, Aluminiumoxid, Siliciumoxid, Alumosilikat, Titandioxid, Zirkoniumdioxid, Magnesiumoxid und/oder Zinkoxid oder deren Gemische eingesetzt.

Bevorzugt werden als Trägermaterialien Feststoffe eingesetzt, die unter Hydrierbedingungen weitgehend inert sind. Dies sind beispielsweise Aktivkohle, Siliciumcarbid, Siliciumdioxid, Titandioxid und/oder Zirkoniumdioxid bzw. Gemische aus diesen Verbindungen. Ganz besonders bevorzugt werden Titandioxide als Trägermaterialien verwendet. Titandioxid tritt in drei Modifikationen auf (Anatas, Rutil, Brookit) auf, von denen Anatas und Rutil die häufigsten sind. Ein bevorzugtes Trägermaterial ist Aerolyst 7711^{®} (Evonik Operations GmbH). Dieses Trägermaterial besteht zu 15 bis 20 Massen-% aus Rutil und zu 80 bis 85 Massen-% aus Anatas. Weitere geeignete Titandioxidträgermaterialien sind beispielsweise solche, die auf Basis von Titanoxiden aus einem Schwefelsäure-Verfahren hergestellt werden. Sie enthalten in der Regel > 98 % Anatas.

Besonders bevorzugt ist, dass als Feststoff-Katalysator für die Hydrierung in Schritt ii. und/oder v. ein Katalysator eingesetzt wird, der Ruthenium als einziges Metall und Titandioxid als Trägermaterial umfasst. In einer bevorzugten Ausführungsform wird der gleiche Katalysator für die Hydrierung in Schritt ii. und in Schritt v. eingesetzt, besonders bevorzugt ist dies ein Katalysator, der Ruthenium als einziges Metall und Titandioxid als Trägermaterial umfasst.

Im erfindungsgemäßen Verfahren wird die Hydrierung in Schritt ii. und/oder v. in flüssiger Phase oder in der Gasphase durchgeführt. Die Hydrierung kann an suspendierten oder stückigen im Festbett angeordneten Katalysatoren kontinuierlich oder diskontinuierlich durchgeführt werden. Im erfindungsgemäßen Verfahren wird eine kontinuierliche Hydrierung an einem im Festbett angeordnetem Katalysator, bei dem sich unter Reaktionsbedingungen die Produkt-/Eduktphase hauptsächlich im flüssigen Zustand befindet, bevorzugt.

Es ist bevorzugt, dass das Hydrieren in Schritt ii. und/oder v. bei einem Druck von 3 bis 300 bar, vorzugsweise 15 bis 200 bar, besonders bevorzugt 50 bis 150 bar, durchgeführt wird.

Weiterhin ist es bevorzugt, dass das Hydrieren in Schritt ii. und/oder iv. bei einer Temperatur von 50°C bis 250°C, vorzugsweise 70 bis 200°C, durchgeführt wird. Diese Temperatur liegt nach erfolgter Hydrierung in den Ausgangsströmen (T₂ und T₄) der Hydriereinheiten vor. Aufgrund der Exothermie der Hydrierreaktion erfolgt die Reaktion nicht bei einer festen Temperatur, sondern in einem Temperaturbereich wie hierin beschrieben. So nimmt die Temperatur des Reaktionsgemisches bei Durchfließen der Hydriereinheit zu. Die Temperatur T₁ ist daher immer kleiner als T₂ und die Temperatur T₃ ist immer kleiner als T₄.

Erfindungsgemäß vorgesehen ist, dass die Temperatur T₁ zumindest zeitweise ungleich der Temperatur T₃ ist, vorzugsweise wobei die Temperatur T₁ höher ist als die Temperatur T₃. "Zumindest zeitweise" bedeutet im Rahmen der vorliegenden Erfindung, dass dieser Zustand (T₁ ≠ T₃, vorzugsweise T₁ > T₃) nicht über das gesamte Hydrierverfahren anhält, sondern nur in oder ab einer bestimmten Zeit vorliegt. Auf der anderen Seite sind damit keine kurzzeitigen Zustandsänderungen gemeint. "Zumindest zeitweise" bedeutet daher, dass dieser Zustand mindestens 30 Minuten lang anhält, vorzugsweise mindestens 2 Stunden lang anhält. Weiterhin kann "zumindest zeitweise" bedeuten, dass mindestens 1% der gesamten Verfahrensdauer durch diesen Zustand gekennzeichnet sind.

Dieser Zustand kann wie folgt realisiert werden: Der Strom A wird mit der Temperatur T₁ über den Eingangsstrom (2b) in den Reaktor geleitet. Aufgrund der Exothermie der Hydrierreaktion nimmt die Temperatur des Reaktionsgemischs im Reaktor zu, vorzugsweise über die Länge des Reaktors. Das erhaltene Gemisch des Produktstroms (3) weist eine Temperatur T₂ auf, die höher ist als T₁.

Ein Teilstrom des erhaltenen Gemischs bzw. des Produktstroms (3) mit der Temperatur T₂ wird zurückgeführt und mit Frisch-Edukt (2a) dem Strom A (2b) zugeleitet. Da Strom A (2b) jedoch eine geringere Temperatur T₁ als T₂ aufweisen sollte, muss das Verfahren so eingerichtet werden, dass diese Bedingung erfüllt wird. Es ließe sich beispielsweise die Temperatur des Frisch-Edukts so einstellen, dass sich beim Mischen mit dem zurückgeführten Teilstrom die Temperatur T₁ einstellt. Es ist im Rahmen der vorliegenden Erfindung jedoch bevorzugt, dass das aus der ersten Hydriereinheit erhaltene Gemisch bzw. der Produktstrom (3) von der Temperatur T₂ heruntergekühlt wird, bevor das Gemisch aufgetrennt wird. Dazu können vorzugsweise bekannte Kühlvorrichtungen eingesetzt werden. Bevorzugt ist der Einsatz eines Wärmetauschers, um die entzogene Wärmeenergie an anderer Stelle in dem Verfahren oder in einem Verbund mehrere Anlagen zu nutzen.

Wird das aus der ersten Hydriereinheit erhaltene Gemisch gekühlt, weisen die beiden Teilströme nach der Auftrennung gleiche Temperatur auf. Die Temperaturen von T₁ und T₃ sind also gleich oder unterscheiden sich um maximal 10 %.

Um den erfindungsgemäßen Zustand (T₁ ≠ T₃, vorzugsweise T₁ > T₃) zu erreichen können in Anbetracht dieser Verfahrensführung unterschiedliche Maßnahmen getroffen werden. Vorzugsweise ist eine weitere Kühlung oder eine Heizung auf Seiten einer der Teilströme vorhanden, um je einen Teilstrom zu kühlen oder einen der Teilströme zu erhitzen. Eine besonders bevorzugte Methode ist im Rahmen der vorliegenden Erfindung die Einrichtung eines Bypasses (4) vor dem Kühler (7) zu dem zur ersten Hydriereinheit geführten Teilstrom. Durch den Bypass kann eine vorher bestimmbare Teilmenge des aus der ersten Hydriereinheit erhaltenen Gemischs bzw. des Produktstroms (3) ungekühlt mit dem zurückzuführenden Teilstrom (6b) kombiniert werden, wodurch die Temperatur T₁ größer ist als die Temperatur T₃ des zur zweiten Hydriereinheit geführten Teilstroms (6c), der komplett über den Kühler (7) geleitet wurde.

Der Vorteil ist, dass die Hydrierung in der ersten Hydriereinheit (1) mit einer höheren Zulauftemperatur T₁ gefahren werden kann, wodurch höhere Hydriertemperaturen möglich sind. Die Aktivitätsverluste des Katalysators über die Zeit werden so ausgeglichen. Gleichzeitig kann die Reaktion in der zweiten Hydriereinheit durchweg bei gleichbleibenden Temperaturverhältnissen betrieben werden. Die Gesamtausbeute und die Güte des Produktstroms bleiben damit konstant.

Im Rahmen der vorliegenden Erfindung, wurde herausgefunden, dass es vorteilhaft ist, wenn der Produktstrom (3) mit der Temperatur T₂ nicht unmittelbar zu der mindestens einen weiteren Hydriereinheit zugeführt wird, sondern gekühlt wird und mit einer geringeren Temperatur T₃ zu der mindestens einen weiteren Hydriereinheit zugeführt wird. Es ist daher besonders bevorzugt, dass die Temperatur T₂ des erhaltenen Gemischs in Schritt iii. höher ist als die Temperatur T₃ des Gemischs, welches in Schritt iv in die Hydriereinheit zugeführt wird.

Weiterhin wurde im Rahmen der vorliegenden Erfindung herausgefunden, dass es vorteilhaft ist, wenn die Temperaturdifferenz ΔT zwischen T₂ und T₃ über den zeitlichen Verlauf des Verfahrens konstant ist. Vorzugsweise ist die Temperaturdifferenz ΔT äquivalent zu der Reaktionsenthalpie der Hydrierreaktion. "Konstant" bedeutet im Rahmen der vorliegenden Erfindung eine maximale Abweichung der Temperatur um ± 10 %, vorzugsweise ± 5 %, besonders bevorzugt ± 1 % bezogen auf die Anfangstemperatur.

Die Temperaturregelung kann dadurch erreicht werden, dass zwischen dem Ausgangsstrom (3) der ersten Hydriereinheit und dem Eingangsstrom (9) der zweiten Hydriereinheit eine Vorrichtung (7) angeordnet ist, welche den Ausgangsstrom (3) kühlt um die gewünschten Temperaturen T₁ und T₃ im Eingangsstrom (2b) zu der ersten Hydriereinheit und im Eingangsstrom (9) zu der mindestens einen weiteren Hydriereinheit zu erreichen. Vorzugsweise wird die Kühlung durch einen Wärmetauscher erreicht. Entsprechende Vorrichtungen sind dem Fachmann geläufig. Dieser kann zum Beispiel im Ausgangsstrom (3) der ersten Hydriereinheit (1), im Eingangsstrom (9) zur zweiten Hydriereinheit oder zwischen den beiden Strömen vorhanden sein. Es kann vorzugsweise ein, zwei, drei oder mehrere Wärmetauscher, verwendet werden.

Es ist im Rahmen des erfindungsgemäßen Verfahrens bevorzugt, dass in Schritt i. ein oder mehrere aromatische Carbonsäureester, vorzugsweise ein oder mehrere aromatische Mono-, Di- und Polycarbonsäureestern bereitgestellt werden.

Im Rahmen des erfindungsgemäßen Verfahrens können aromatische Verbindungen, wie aromatische Poly- und/oder Monocarbonsäuren oder deren Derivate, insbesondere deren Alkylester zu den entsprechenden alicyclischen Polycarbonsäureverbindungen umgesetzt werden. Dabei können sowohl Vollester als auch Partialester hydriert werden. Unter Vollester wird eine Verbindung verstanden, bei der alle Säuregruppen verestert sind. Partialester sind Verbindungen mit mindestens einer freien Säuregruppe (oder ggf. einer Anhydridgruppe) und mindestens einer Estergruppe.

Werden im erfindungsgemäßen Verfahren Polycarbonsäureester eingesetzt, so enthalten diese bevorzugt 2, 3 oder 4 Esterfunktionen.

Es ist im Rahmen des erfindungsgemäßen Verfahrens bevorzugt, dass in Schritt i. ein oder mehrere Benzol-, Diphenyl-, Naphthalin-, Diphenyloxid, Anthracen-Di- oder Polycarbonsäureester bereitgestellt werden. Die mit dem erfindungsgemäßen Verfahren erhaltenen alicyclischen Polycarbonsäuren bzw. deren Derivate bestehen aus einem oder mehreren, ggf. durch eine C-C-Bindung verknüpfte oder ankondensierte C₆-Ringe.

Weiterhin ist es bevorzugt, dass in Schritt i. ein oder mehrere aromatische Carbonsäureester mit einer Alkoholkomponente ausgewählt aus der Gruppe bestehend aus verzweigten oder unverzweigten Alkoxyalkyl-, Cycloalkyl- und/oder Alkylgruppen mit 1 bis 25 Kohlenstoffatomen, vorzugsweise C₈-C₁₀-Phthalat, C₈-C₁₀-Terephthalat, C₈-C₁₀-Isophthalat und C₈-C₁₀-Trimellitat, besonders bevorzugt Di-2-ethylhexylphthalat, Di-isononylphthalat, Di-2-ethylhexylterephthalat, Di-isononylterephthalat, Di-2-ethylhexylisophthalat, Tri-isononylisophthalat, Di-2-ethylhexyltrimellitat und Tri-isononyltrimellitat, bereitgestellt werden.

Hierbei bedeutet C₈ vorzugsweise 2-Ethylhexl oder n-Octyl, C₉ bdeutet Isononyl und C₁₀ bedeutet Isodecyl oder 2-Propylheptyl.

Vorzugsweise ist das Verfahren ein Verfahren zur Hydrierung von 1,2-; 1,3- oder 1,4-Benzoldicarbonsäureester, und/oder der 1,2,3-; 1,2,4- oder 1,3,5-Benzoltricarbonsäureester, d. h. es werden die Isomere der 1,2-; 1,3- oder 1,4-Cyclohexandicarbonsäureester, oder der 1,2,3-; 1,3,5- oder 1,2,4-Cyclohexantricarbonsäureester gebildet.

Im erfindungsgemäßen Verfahren können beispielsweise Ester folgender aromatischer Carbonsäuren eingesetzt werden: 1,2-Naphthalindicarbonsäure, 1,3-Naphthalindicarbonsäure, 1,4-Naphthalindicarbonsäure, 1,5-Naphthalindicarbonsäure, 1,6-Naphthalindicarbonsäure, 1,7-Naphthalindicarbonsäure, 1,8-Naphthalindicarbonsäure, Phthalsäure (Benzol-1,2-dicarbonsäure), Isophthalsäure (Benzol-1,3-dicarbonsäure), Terephthalsäure (Benzol-1,4-dicarbonsäure), Benzol-1,2,3-tricarbonsäure, Benzol-1,2,4-tricarbonsäure (Trimellitsäure), Benzol-1,3,5-tricarbonsäure (Trimesinsäure), Benzol-1,2,4,5-tetracarbonsäure (Pyromellitsäure). Weiterhin können Säuren eingesetzt werden, die aus den genannten Säuren durch Substitution eines oder mehrerer am aromatischen Kern gebundenen Wasserstoffatome durch Alkyl-, Cycloalkyl- oder Alkoxyalkylgruppen entstehen.

Vorzugsweise werden Alkyl-, Cycloalkyl- sowie Alkoxyalkylester z. B. der oben genannten Säuren eingesetzt, wobei diese Reste unabhängig voneinander 1 bis 25, insbesondere 3 bis 15, ganz besonders 8 bis 13 C-Atome, insbesondere 9 C-Atome, umfassen. Diese Reste können linear oder verzweigt sein. Hat ein Edukt mehr als eine Estergruppe, dann können diese Reste gleich oder verschieden sein.

Im erfindungsgemäßen Verfahren können als Ester einer aromatischen Polycarbonsäure beispielsweise folgende Verbindungen eingesetzt werden: Terephthalsäuremonomethylester, Terephthalsäuredimethylester, Terephthalsäurediethylester, Terephthalsäuredi-n-propylester, Terephthalsäuredibutylester, Terephthalsäurediisobutylester, Terephthalsäuredi-tert.-butylester, Terephthalsäure-dipentylester, Terephthalsäuremonoglykolester, Terephthalsäurediglykolester, Terephthalsäure-n-octylester, Terephthalsäurediisooctylester, Terephthalsäuredi-2-ethylhexylester, Terephthalsäuredi-n-nonylester, Terephthalsäurediisononylester, Terephthalsäure-di-2-propylheptylester, Terephthalsäuredi-n-decylester, Terephthalsäuredi-n-undecylester, Terephthalsäurediisodecylester, Terephthalsäurediisododecylester, Terephthalsäure-ditridecylester, Terephthalsäuredi-n-octadecylester, Terephthalsäurediisooctadecylester, Terephthalsäuredi-n-eicosylester, Terephthalsäuremonocyclohexylester; Phthalsäuremonomethylester, Phthalsäuredimethylester, Phthalsäuredi-n-propylester, Phthalsäuredi-n-butylester, Phthalsäurediisobutylester, Phthalsäuredi-tert.-butylester, Phthalsäuremonoglykolester, Phthalsäurediglykolester, Phthalsäuredi-n-octylester, Phthalsäurediisooctylester, Phthalsäuredi-2-ethylhexylester, Phthalsäuredi-n-nonylester, Phthalsäurediisononylester, Phthalsäuredi-n-decylester, Phthalsäuredi-2-propylheptylester, Phthalsäurediisodecylester, Phthalsäuredi-n-undecylester, Phthalsäurediisoundecylester, Phthalsäureditridecylester, Phthalsäuredi-n-octadecylester, Phthalsäurediisooctadecylester, Phthalsäuredi-n-eicosylester, Phthalsäuremonocyclohexylester; Phthalsäuredicyclohexylester, Isophthalsäuremonomethylester, Isophthalsäuredimethylester, Isophthalsäurediethylester, Isophthalsäuredi-n-propylester, Isophthalsäuredi-n-butylester, Isophthalsäurediisobutylester, Isophthalsäuredi-tert.-butylester, Isophthalsäuremonoglykolester. Isophthalsäurediglykolester, Isophthalsäuredi-n-octylester, Isophthalsäurediisooctylester, Isophthalsäuredi-2-ethylhexylester, Isophthalsäuredi-n-nonylester, Isophthalsäurediisononylester, Isophthalsäuredi-n-decylester, Isophthalsäurediisodecylester, Isophthalsäuredi-n-undecylester, Isophthalsäurediisododecylester, Isophthalsäuredi-n-dodecylester, Isophthalsäureditridecylester, Isophthalsäuredi-n-octadecylester, Isophthalsäurediisooctadecylester, Isophthalsäuredi-n-eicosylester, Isophthalsäuremonocyclohexylester.

Das erfindungsgemäße Verfahren ist prinzipiell auch auf Benzoesäure und deren Ester anwendbar. Hierunter werden neben Benzoesäurealkylester auch Benzoate von Diolen, wie beispielsweise Glycoldibenzoat, Diethylenglycolbenzoat, Triethylenglycoldibenzoat oder Propylenglycoldibenzoat, verstanden. Die Alkoholkomponente der Benzoesäurealkylester kann aus 1 bis 25, bevorzugt 8 bis 13 Kohlenstoffatomen, jeweils linear oder verzweigt, bestehen.

Großtechnisch werden vorzugsweise aromatische Ester, insbesondere Vollester häufig aus Alkoholgemischen hergestellt. Entsprechende Alkoholgemische sind beispielsweise: Cs-Alkoholgemische, hergestellt aus linearen Butenen durch Hydroformylierung und anschließender Hydrierung; Cs-Alkoholgemische, hergestellt aus Butengemischen, die lineare Butene und Isobuten enthalten, durch Hydroformylierung und anschließende Hydrierung; Cs-Alkoholgemische, hergestellt aus einem Penten oder aus einem Gemisch von zwei oder mehreren Pentenen, durch Hydroformylierung und anschließende Hydrierung; C₇-Alkoholgemische, hergestellt aus der Trimerisierung von Ethylen oder Dimerisierung von Propylen oder einem Hexenisomer oder einem sonstigen Gemisch von Hexenisomeren, durch Hydroformylierung und anschließende Hydrierung; C₈-Alkoholgemische, wie 2-Ethylhexanol (2 Isomere), hergestellt durch Aldolkondensation von n-Butyraldehyd und anschließende Hydrierung; Cs-Alkoholgemische, hergestellt aus C₄-Olefinen durch Dimerisierung, Hydroformylierung und Hydrierung. Dabei kann zur Herstellung der C₉-Alkohole von Isobuten oder von einem Gemisch linearer Butene oder von Gemischen mit linearen Butenen und Isobuten ausgegangen werden. Die C₄-Olefine können mit Hilfe unterschiedlicher Katalysatoren dimerisiert werden, wie beispielsweise Protonensäuren, Zeolithe, metallorganische Nickelverbindungen oder feste nickelhaltige Kontakte. Die Hydroformylierung der C₈-Olefingemische kann mit Hilfe von Rhodium- oder Kobaltkatalysatoren erfolgen. Es gibt daher eine Vielzahl von technischen C₉-Alkoholgemischen; C₁₀-Alkoholgemische, hergestellt aus Tripropylen durch Hydroformylierung und anschließende Hydrierung; 2-Propylheptanol (2 Isomere), hergestellt durch Aldolkondensation von Valeraldehyd und anschließende Hydrierung; C₁₀-Alkoholgemische, hergestellt aus einem Gemisch von mindestens zwei Cs-Aldehyden durch Aldolkondensation und anschließende Hydrierung; C₁₃-Alkohollgemische, hergestellt aus Hexaethylen, Tetrapropylen oder Tributen durch Hydroformylierung und anschließende Hydrierung.

Weitere Alkoholgemische können durch Hydroformylierung und anschließende Hydrierung aus Olefinen bzw. Olefingemischen gewonnen werden, die beispielsweise bei Fischer-Tropsch-Synthesen, bei Dehydrierungen von Kohlenwasserstoffen, Metathesereaktionen, beim Polygasverfahren oder anderen technischen Prozessen anfallen. Darüber hinaus können auch Olefingemische mit Olefinen unterschiedlicher C-Zahlen für die Herstellung von Alkoholgemischen eingesetzt werden.

Im erfindungsgemäßen Verfahren können alle Estergemische, hergestellt aus aromatischen Polycarbonsäuren und den oben genannten Alkoholgemischen, eingesetzt werden. Erfindungsgemäß werden bevorzugt Ester, hergestellt aus Phthalsäure oder Phthalsäureanhydrid sowie Terephthalsäure oder Dimethylterephthalat und einem Gemisch isomerer Alkohole mit 4 bis 13 C-Atomen, eingesetzt.

Bevorzugt ist ein Verfahren zur Herstellung von einer oder mehrerer alicyclischer Verbindungen, umfassend die Schritte:
i. Bereitstellen eines Stroms A (2b), umfassend eine oder mehrere aromatische Verbindungen ausgewählt aus der Gruppe bestehend aus Estern der Phthalsäure, Isophthalsäure, Terephthalsäure und/oder Trimellitsäure, besonders bevorzugt ausgewählt aus der Gruppe bestehend aus Terephthalsäuredipentylester, Terephthalsäuredi-2-ethylhexylester, Terephthalsäurediisononylester, Phthalsäuredipentylester, Phthalsäuredi-2-ethylhexylester, Phthalsäurediisononylester, Isophthalsäuredipentylester, Isophthalsäuredi-2-ethylhexylester, Isophthalsäurediisononylester, Trimellitsäuretripentylester, Trimellitsäuretri-2-ethylhexylester, Trimellitsäuretriisononylester, oder Mischungen daraus,
   und ein wasserstoffhaltiges Hydriergas;
ii. Zuführen des Stroms A (2b) mit einer Temperatur T₁ zu einer ersten Hydriereinheit (1) und Hydrieren der einen oder mehreren aromatischen Verbindungen zu einer oder mehrerer entsprechender alicyclischer Verbindungen;
iii. Erhalten eines Gemisches als ersten Produktstrom (3) mit einer Temperatur T₂ umfassend aromatische Verbindungen und alicyclische Verbindungen ausgewählt aus der Gruppe bestehend aus 1,2-Dialkylcyclohexandicarbonsäureester, 1,3-Dialkylcyclohexandicarbonsäureester, 1,4-Dialkylcyclohexandicarbonsäureestern und 1,2,4-Trimellitsäureester, besonders bevorzugt ausgewählt aus der Gruppe bestehend aus 1,4-Cyclohexandicarbonsäuredipentylester, 1,4-Cyclohexandicarbonsäuredi-2-ethylhexylester, 1,4-Cyclohexandicarbonsäurediisononylester, 1,2-Cyclohexandicarbonsäuredipentylester, 1,2-Cyclohexandicarbonsäuredi-2-ethylhexylester, 1,2-Cyclohexandicarbonsäurediisononylester, 1,3-Cyclohexandicarbonsäuredipentylester, 1,3-Cyclohexandicarbonsäuredi-2-ethylhexylester, 1,3-Cyclohexandicarbonsäurediisononylester, 1,2,4-Cyclohexantricarbonsäuretripentylester, 1,2,4-Cyclohexantricarbonsäuretri-2-ethylhexylester, 1,2,4-Cyclohexantricarbonsäuretriisononylester;
iv. Auftrennen des in Schritt iii. erhaltenen Produktstroms (3) in einen Teilstrom (8), der zu dem Strom A (2b) in Schritt i. zugeführt und als Teil des Stroms A (2b) in Schritt ii. erneut hydriert wird, sowie einen Teilstrom (6c), der als Strom B (9) bei einer Temperatur T₃ in eine oder mehrere weitere Hydriereinheiten (11) zugeführt wird;
v. Hydrieren der in Strom B (9) enthaltenen aromatischen Verbindungen wie in Schritt i. definiert zu den entsprechenden alicyclischen Verbindungen in der einen oder mehreren weiteren Hydriereinheiten (11), und
vi. Erhalten eines Produktstroms (12) mit einer Temperatur T₄ umfassend eine oder mehrere alicyclische Verbindungen ausgewählt aus der Gruppe bestehend aus 1,2-Dialkylcyclohexandicarbonsäureester, 1,3-Dialkylcyclohexandicarbonsäureester, 1,4-Dialkylcyclohexandicarbonsäureestern und 1,2,4-Trimellitsäureester, besonders bevorzugt ausgewählt aus der Gruppe bestehend aus 1,4-Cyclohexandicarbonsäuredipentylester, 1,4-Cyclohexandicarbonsäuredi-2-ethylhexylester, 1,4-Cyclohexandicarbonsäurediisononylester, 1,2-Cyclohexandicarbonsäuredipentylester, 1,2-Cyclohexandicarbonsäuredi-2-ethylhexylester, 1,2-Cyclohexandicarbonsäurediisononylester, 1,3-Cyclohexandicarbonsäuredipentylester, 1,3-Cyclohexandicarbonsäuredi-2-ethylhexylester, 1,3-Cyclohexandicarbonsäurediisononylester, 1,2,4-Cyclohexantricarbonsäuretripentylester, 1,2,4-Cyclohexantricarbonsäuretri-2-ethylhexylester, 1,2,4-Cyclohexantricarbonsäuretriisononylester entsprechend zu den in Schritt i. bereitgestellten einen oder mehreren aromatischen Verbindungen,
wobei zumindest zeitweise die Temperatur T₁ ungleich der Temperatur T₃ ist, vorzugsweise wobei die Temperatur T₁ höher ist als die Temperatur T₃.

Die im Produktstrom (3) enthaltenen alicyclischen Verbindungen sind abhängig von dem eingesetzten Edukt. So wird beispielsweise 1,2-Cyclohexandicarbonsäurediisononylester als Produkt enthalten, wenn Phthalsäurediisononylester als Edukt eingesetzt werden.

Besonders bevorzugt ist ein Verfahren zur Herstellung von einer oder mehrerer alicyclischer Verbindungen, umfassend die Schritte:
i. Bereitstellen eines Stroms A (2b) enthaltend Phthalsäurediisononylester (DINP) oder Phthalsäuredi-2-ethylhexylester (DEHP) und ein wasserstoffhaltiges Hydriergas;
ii. Zuführen des Stroms A (2b) mit einer Temperatur T₁ zu einer ersten Hydriereinheit (1) und Hydrieren der einen oder mehreren aromatischen Verbindungen zu einer oder mehrerer entsprechender alicyclischer Verbindungen;
iii. Erhalten eines Gemisches als ersten Produktstrom (3) mit einer Temperatur T₂ umfassend 1,2-Cyclohexandicarbonsäurediisononylester (DINCH) oder 1,2-Cyclohexandicarbonsäuredi-2-ethylhexylester (DEHCH);
iv. Auftrennen des in Schritt iii. erhaltenen Produktstroms (3) in einen Teilstrom (8), der zu dem Strom A (2b) in Schritt i. zugeführt und als Teil des Stroms A (2b) in Schritt ii. erneut hydriert wird, sowie einen Teilstrom (6c), der als Strom B (9) bei einer Temperatur T₃ in eine oder mehrere weitere Hydriereinheiten (11) zugeführt wird;
v. Hydrieren der in Strom B (9) enthaltenen aromatischen Verbindungen zu den entsprechenden alicyclischen Verbindungen in der einen oder mehreren weiteren Hydriereinheiten (11), und
vi. Erhalten eines Produktstroms (12) mit einer Temperatur T₄ umfassend 1,2-Cyclohexandicarbonsäurediisononylester (DINCH) oder 1,2-Cyclohexandicarbonsäuredi-2-ethylhexylester (DEHCH),
wobei zumindest zeitweise die Temperatur T₁ ungleich der Temperatur T₃ ist, vorzugsweise wobei die Temperatur T₁ höher ist als die Temperatur T₃.

Besonders bevorzugt ist weiterhin ein Verfahren zur Herstellung von einer oder mehrerer alicyclischer Verbindungen, umfassend die Schritte:
i. Bereitstellen eines Stroms A (2b) enthaltend Terephthalsäurediisononylester oder Terephthalsäuredi-2-ethylhexylester und ein wasserstoffhaltiges Hydriergas;
ii. Zuführen des Stroms A (2b) mit einer Temperatur T₁ zu einer ersten Hydriereinheit (1) und Hydrieren der einen oder mehreren aromatischen Verbindungen zu einer oder mehrerer entsprechender alicyclischer Verbindungen;
iii. Erhalten eines Gemisches als ersten Produktstrom (3) mit einer Temperatur T₂ umfassend 1,4-Cyclohexandicarbonsäurediisononylester oder 1,4-Cyclohexandicarbonsäuredi-2-ethylhexylester;
iv. Auftrennen des in Schritt iii. erhaltenen Produktstroms (3) in einen Teilstrom (8), der zu dem Strom A (2b) in Schritt i. zugeführt und als Teil des Stroms A (2b) in Schritt ii. erneut hydriert wird, sowie einen Teilstrom (6c), der als Strom B (9) bei einer Temperatur T₃ in eine oder mehrere weitere Hydriereinheiten (11) zugeführt wird;
v. Hydrieren der in Strom B (9) enthaltenen aromatischen Verbindungen zu den entsprechenden alicyclischen Verbindungen in der einen oder mehreren weiteren Hydriereinheiten (11), und
vi. Erhalten eines Produktstroms (12) mit einer Temperatur T₄ umfassend 1,4-Cyclohexandicarbonsäurediisononylester oder 1,4-Cyclohexandicarbonsäuredi-2-ethylhexylester,
wobei zumindest zeitweise die Temperatur T₁ ungleich der Temperatur T₃ ist, vorzugsweise wobei die Temperatur T₁ höher ist als die Temperatur T₃.

Besonders bevorzugt ist weiterhin ein Verfahren zur Herstellung von einer oder mehrerer alicyclischer Verbindungen, umfassend die Schritte:
i. Bereitstellen eines Stroms A (2b) enthaltend Trimellitsäuretriisononylester (TINTM) oder Trimellitsäuretri-2-ethylhexylester (TOTM) und ein wasserstoffhaltiges Hydriergas;
ii. Zuführen des Stroms A (2b) mit einer Temperatur T₁ zu einer ersten Hydriereinheit (1) und Hydrieren der einen oder mehreren aromatischen Verbindungen zu einer oder mehrerer entsprechender alicyclischer Verbindungen;
iii. Erhalten eines Gemisches als ersten Produktstrom (3) mit einer Temperatur T₂ umfassend 1,2,4-Cyclohexantricarbonsäuretriisononylester oder 1,2,4-Cyclohexantricarbonsäuretri-2-ethylhexylester;
iv. Auftrennen des in Schritt iii. erhaltenen Produktstroms (3) in einen Teilstrom (8), der zu dem Strom A (2b) in Schritt i. zugeführt und als Teil des Stroms A (2b) in Schritt ii. erneut hydriert wird, sowie einen Teilstrom (6c), der als Strom B (9) bei einer Temperatur T₃ in eine oder mehrere weitere Hydriereinheiten (11) zugeführt wird;
v. Hydrieren der in Strom B (9) enthaltenen aromatischen Verbindungen zu den entsprechenden alicyclischen Verbindungen in der einen oder mehreren weiteren Hydriereinheiten (11), und
vi. Erhalten eines Produktstroms (12) mit einer Temperatur T₄ umfassend 1,2,4-Cyclohexantricarbonsäuretriisononylester oder 1,2,4-Cyclohexantricarbonsäuretri-2-ethylhexylester,
wobei zumindest zeitweise die Temperatur T₁ ungleich der Temperatur T₃ ist, vorzugsweise wobei die Temperatur T₁ höher ist als die Temperatur T₃.

Das erfindungsgemäße Verfahren wird vorzugsweise unter folgenden Bedingungen durchgeführt:
Im Zulauf der ersten Hydriereinheit (Schlaufenfahrweise) liegt die Konzentration der aromatischen Verbindungen als Edukt zwischen 5 und 30 Massen-%, insbesondere zwischen 8 und 15 Massen-%. Im Ausgangstrom (3) der ersten Hydriereinheit liegt die Konzentration des Edukts zwischen 0,3 und 8 Massen-%, insbesondere zwischen 1,5 und 4 Massen-%. Die spezifische Katalysatorbelastung (LHSV, Liter Frisch-Edukt je Liter Katalysatorje Stunde) in der ersten Hydriereinheit (1) beträgt 0,1 bis 5, h⁻¹ insbesondere 0,5 bis 3 h⁻¹.

Die Oberflächenbelastung in der ersten Hydriereinheit (1) liegt im Bereich von 25 bis 140 m³/m²/h, insbesondere im Bereich von 50 bis 90 m³/m²/h.

Die durchschnittlichen Hydriertemperaturen in der ersten Hydriereinheit (1) sind 70 bis 150 °C, insbesondere 80 bis 120 °C.

Der Hydrierdruck in der ersten Hydriereinheit (1) beträgt 25 bis 200 bar, insbesondere 80 bis 110 bar.

Die spezifische Katalysatorbelastung der zweiten Hydriereinheit (11) (Liter Edukt je Liter Katalysatorje Stunde) beträgt 1 bis 8 h⁻¹, insbesondere 2 bis 5 h⁻¹.

In der zweiten Hydriereinheit (11) liegt die durchschnittliche Temperatur zwischen 70 und 150 °C, insbesondere 80 und 120 °C.

Der Hydrierdruck in der zweiten Hydriereinheit beträgt 25 bis 200 bar, insbesondere 80 bis 100 bar.

Die Verfahrensvarianten eigenen sich insbesondere zur Hydrierung von Phthalsäureestern, besonders für Di-isononylphthalate (als Isomerengemisch "Di-isononylphthalat" z. B. VESTINOL 9 der OXENO GmbH) oder Di-2-ethylhexylphthalat.

Ein weiterer Aspekt der vorliegenden Erfindung ist die Bereitstellung einer Vorrichtung zum Durchführen eines erfindungsgemäßen Verfahrens, umfassend eine erste Hydriereinheit (1) und eine oder mehrere weitere Hydriereinheiten (11) und einen oder mehrere Wärmetauscher (7), wobei der Wärmetauscher (7) so angeordnet ist, dass der Ausgangsstrom (3) über einen Eingangsstroms (5) in den Wärmetauscher (7) geleitet wird und der Ausgangsstrom des Wärmetauschers (6a) eine geringere Temperatur als der Eingangsstrom (5) aufweist und anschließend über Rohrleitungen (6b, 6c) als Eingangsstrom (9) in eine weitere Hydriereinheit (11) zugeführt wird und/oder über den Strom (10) in wiederum eine oder mehrere weitere Hydriereinheiten (11) zugeführt wird und/oder über den Strom 8 dem Eingangsstrom zu der ersten Hydriereinheit (1) zugeführt wird, vorzugsweise wobei die Ströme 3 und 9 eine unterschiedliche Temperatur besitzen.

Vorzugsweise kann mindestens ein weiterer Wärmetauscher vorliegen, welcher zum Beispiel im Eingangsstrom (9) der mindestens einen weiteren Hydriereinheit (11), angeordnet ist.

Es ist bevorzugt, dass im Rahmen der erfindungsgemäßen Vorrichtung die erste und/oder mindestens eine der weiteren Hydriereinheiten (1,11) einen oder mehrere Festbettkatalysatoren aufweist, vorzugsweise wobei der Feststoff-Katalysator mindestens ein Metall der achten Nebengruppe des Periodensystems der Elemente, besonders bevorzugt Ruthenium, aufweist. Bevorzugtes Trägermaterial ist Titandioxid. Es gilt das für die verwendeten Katalysatoren hierin Gesagte entsprechend.

Es ist ebenfalls bevorzugt, dass die Rohrleitungen der ersten Hydriereinheit (1) so angeordnet sind, dass der Ausgangsstrom (3) der ersten Hydriereinheit (1) über einen Bypass (4) oder über die Rohrleitungen (6b,8) als Eingangsstrom (2b) zu der ersten Hydriereinheit (1) rezykliert werden kann. Die erste Hydriereinheit (1) wird dann in Schlaufenfahrweise betrieben.

Weiterhin ist es bevorzugt, in der ersten und/oder in einer der weiteren Hydriereinheiten eine Mischung aus aromatischen Verbindungen und entsprechenden alizyklischen Verbindungen, vorzugsweise eine Mischung aus aromatische Carbonsäureester und deren entsprechenden alizyklischen Verbindungen mit einer Alkoholkomponente ausgewählt aus der Gruppe bestehend aus verzweigten oder unverzweigten Alkoxyalkyl-, Cycloalkyl- und/oder Alkylgruppen mit 1 bis 25 Kohlenstoffatomen, vorzugsweise aus C₈-C₁₀-Phthalat, C₈-C₁₀-Terephthalat, C₈-C₁₀-Isophthalat und C₈-C₁₀-Trimellitat, besonders bevorzugt Di-2-ethylhexylphthalat, Di-isononylphthalat, Di-2-ethylhexylterephthalat, Di-isononylterephthalat, Di-2-ethylhexylisophthalat, Tri-isononylisophthalat, Di-2-ethylhexyltrimellitat und Tri-isononyltrimellitat, Diisononylphthalat und/oder Didecylphthalat und Di-isononyl-cyclohexandicarbonsäureester und/oder Di-decyl-cyclohexandicarbonsäureester und deren entsprechender alizyklischen Verbindungen, vorhanden ist. Es gilt das hierin für die Edukte (aromatische Verbindungen) und Produkte (alicyclische Verbindungen) Gesagte entsprechend.

Bevorzugt im Rahmen der vorliegenden Erfindung, ist die Verwendung der erfindungsgemäß hergestellten alicyclischen Polycarbonsäureester als Weichmacher in Kunststoffen. Bevorzugte Kunststoffe sind PVC, Homo- und Copolymere auf Basis von Ethylen, Propylen, Butadien, Vinylacetat, Glycidylacrylat, Glycidylmethacrylat, Acrylaten, Acrylaten mit am Sauerstoffatom der Estergruppe gebundenen Alkylresten von verzweigten oder unverzweigten Alkoholen mit einem bis zehn Kohlenstoffatome(n), Styrol, Acrylnitril, Homo- oder Copolymere von cyclischen Olefinen.

Neben den obengenannten Anwendungen können die erfindungsgemäß hergestellten alicyclischen Polycarbonsäureester als Schmierölkomponente, als Bestandteil von Kühlflüssigkeiten und Metallbearbeitungsflüssigkeiten verwendet werden. Ebenso können sie als Komponente in Farben, Lacken, Tinten und Klebstoffen eingesetzt werden.

Die folgenden Beispiele sollen die Erfindung erläutern, ohne deren Anwendungsbreite, die sich aus der Beschreibung und den Patentansprüchen ergibt, einzuschränken.

### Beispiele:

In den nachfolgenden Beispielen wird die Durchführung der Hydrierung des Phthalsäurediisononylesters (DINP) zum 1,2-Cyclohexandicarbonsäurediisononylester (DINCH) beschrieben, wobei eine Endkonzentration von DINP im Ausgang der letzten Stufe von kleiner als 0,05 Gew.-% der Eingangskonzentration in der 1. Stufe erreicht werden sollte.

Alle Beispiele wurden mittels der Software Aspen V10 anhand echter Prozessdaten und darauf basierenden kinetischen Modellen simuliert. Die Hydriereinheit bestand aus einem Reaktor, der in Schlaufenfahrweise kontinuierlich betrieben wurde, und einem sich anschließenden Reaktor, der im geraden Durchgang betrieben wurde. Bei allen Versuchen strömen die flüssige Phase und das Hydriergas im Gleichstrom von oben nach unten.

Entsprechend der Figur 1 werden die Eingangstemperatur des ersten Reaktors als T₁ (2b), die Ausgangstemperatur des zweiten Reaktors als T₂ (3), die Eingangstemperatur des dritten Reaktors als T₃ (9) und die Ausgangstemperatur des zweiten Reaktors als T₄ (12) bezeichnet.

Wird die Reaktion gestartet und nimmt man eine Katalysatoraktivität von 100% und volle Anlagenlast an, ergeben sich folgende Temperaturen:

| 100% Katalysatoraktivität, 100% Anlagenlast | | | |
|---|---|---|---|
| T₁ / °C | T₂ / °C | T₃ / °C | T₄ / °C |
| 101,7 | 115,5 | 107,4 | 140,7 |

Mit der Zeit sinkt die Aktivität des Katalysators ab. Nimmt man eine Katalysatoraktivität von 70% und volle Anlagenlast an, ergeben sich folgende Temperaturen:

| 70% Katalysatoraktivität, 100% Anlagenlast | | | |
|---|---|---|---|
| T₁ / °C | T₂ / °C | T₃ / °C | T₄ / °C |
| 104,5 | 117,6 | 110,4 | 152,6 |

Die Temperatur am Eingang muss gesteigert werden, um weiterhin die Endkonzentration an DINP von kleiner 0,05 Gew.-% erreichen zu können.

### Beispiel 1 (Vergleichsbeispiel)

Mit fortschreitender Zeit sinkt die Aktivität des Katalysators weiter ab. Nimmt man eine Katalysatoraktivität von 30% und volle Anlagenlast an, ergeben sich folgende Temperaturen:

| 30% Katalysatoraktivität, 100% Anlagenlast | | | |
|---|---|---|---|
| T₁ / °C | T₂ / °C | T₃ / °C | T₄ / °C |
| 122,3 | 134,8 | 125,4 | 172,0 |

Um die Endkonzentration an DINP von kleiner 0,05 Gew.-% einhalten zu können, muss die Eingangstemperatur T₁ weiter gesteigert werden. Aufgrund der entstehenden Reaktionswärme steigt die Temperatur T₄ soweit an, dass die Anlage über die maximal erlaubte Temperatur von 160 °C steigt. Die Anlage muss abgeschaltet werden. Eine andere Möglichkeit wäre eine Reduktion der Anlagenlast, was jedoch erheblich geringere Produktionsmengen zur Folge hätte.

### Beispiel 2 (erfindungsgemäßes Beispiel)

Beispiel 2 wurde mit der erfindungsgemäßen Temperaturregelung durchgeführt, bei der T₁ und T₃ unabhängig voneinander eingestellt werden können und Temperatur T₁ ungleich der Temperatur T₃ ist. Nimmt man eine Katalysatoraktivität von 30% und volle Anlagenlast an, ergeben sich folgende Temperaturen:

| 30% Katalysatoraktivität, 100% Anlagenlast | | | |
|---|---|---|---|
| T₁ / °C | T₂ / °C | T₃ / °C | T₄ / °C |
| 122,3 | 134,8 | 108,1 | 155,2 |

Es zeigt sich, dass die Temperatur in einem akzeptablen Bereich ist und weder eine Anlagenabschaltung droht noch die Anlagenlast reduziert werden muss.

## Patentansprüche

1. Verfahren zur Herstellung von einer oder mehrerer alicyclischer Verbindungen, umfassend die Schritte:
i. Bereitstellen eines Stroms A (2b), umfassend eine oder mehrere aromatische Verbindungen und eines wasserstoffhaltigen Hydriergases;
ii. Zuführen des Stroms A (2b) mit einer Temperatur T₁ zu einer ersten Hydriereinheit (1) und Hydrieren der einen oder mehreren aromatischen Verbindungen zu einer oder mehrerer entsprechender alicyclischer Verbindungen;
iii. Erhalten eines Gemisches als ersten Produktstrom (3) mit einer Temperatur T₂ umfassend aromatische Verbindungen und alicyclische Verbindungen;
iv. Auftrennen des in Schritt iii. erhaltenen Produktstroms (3) in einen Teilstrom (8), der zu dem Strom A (2b) in Schritt i. zugeführt und als Teil des Stroms A (2b) in Schritt ii. erneut hydriert wird, sowie einen Teilstrom (6c), der als Strom B (9) bei einer Temperatur T₃ in eine oder mehrere weitere Hydriereinheiten (11) zugeführt wird;
v. Hydrieren der in Strom B (9) enthaltenen aromatischen Verbindungen zu den entsprechenden alicyclischen Verbindungen in der einen oder mehreren weiteren Hydriereinheiten (11), und
vi. Erhalten eines Produktstroms (12) mit einer Temperatur T₄ umfassend eine oder mehrere alicyclische Verbindungen entsprechend zu den in Schritt i. bereitgestellten einen oder mehreren aromatischen Verbindungen,
wobei zumindest zeitweise die Temperatur T₁ ungleich der Temperatur T₃ ist, vorzugsweise wobei die Temperatur T₁ höher ist als die Temperatur T₃.

2. Verfahren gemäß Anspruch 1, wobei das Hydrieren der in Schritt i. bereitgestellten aromatischen Verbindungen an einem oder mehreren in einem Festbett der Hydriereinheiten angeordneten Feststoff-Katalysatoren mit dem in Schritt i. bereitgestellten wasserstoffhaltigen Gas erfolgt.

3. Verfahren gemäß Anspruch 1 oder 2, wobei der Feststoff-Katalysator mindestens ein Metall der achten Nebengruppe des Periodensystems der Elemente, vorzugsweise Ruthenium, aufweist.

4. Verfahren gemäß einem der vorangehenden Ansprüche, wobei Schritt iv. mit einem Kreislaufverhältnis von 1 zu 10 bis 1 zu 50, vorzugsweise von 1 zu 10 bis 1 zu 40, besonders bevorzugt von 1 zu 30 durchgeführt wird.

5. Verfahren gemäß einem der vorangehenden Ansprüche, wobei das Hydrieren in Schritt ii. und/oder v. bei einem Druck von 3 bis 300 bar, vorzugsweise 15 bis 200 bar, besonders bevorzugt 100 bis 200 bar, durchgeführt wird.

6. Verfahren gemäß einem der vorangehenden Ansprüche, wobei das Hydrieren in Schritt ii. und/oder v. bei einer Temperatur von 50°C bis 250°C, vorzugsweise 100 bis 200°C, durchgeführt wird.

7. Verfahren gemäß einem der vorangehenden Ansprüche, wobei die Temperatur T₂ des erhaltenen Gemischs in Schritt iii. höher ist als die Temperatur T₃ des Gemischs, welches in Schritt iv in die Hydriereinheit zugeführt wird.

8. Verfahren gemäß Anspruch 7, wobei die Temperaturdifferenz ΔT zwischen T₂ und T₃ über den zeitlichen Verlauf der Durchführung des Verfahrens konstant ist.

9. Verfahren gemäß einem der vorangehenden Ansprüche, wobei in Schritt i. ein oder mehrere aromatische Carbonsäureester, vorzugsweise ein oder mehrere aromatische Mono-, Di- und Polycarbonsäureestern bereitgestellt werden.

10. Verfahren gemäß einem der vorangehenden Ansprüche, wobei in Schritt i. ein oder mehrere Benzol-, Diphenyl-, Naphthalin-, Diphenyloxid, Anthracen-Di- oder Polycarbonsäureester bereitgestellt werden.

11. Verfahren gemäß einem der vorangehenden Ansprüche, wobei in Schritt i. ein oder mehrere aromatische Carbonsäureester mit einer Alkoholkomponente ausgewählt aus der Gruppe bestehend aus verzweigten oder unverzweigten Alkoxyalkyl-, Cycloalkyl- und/oder Alkylgruppen mit 1 bis 25 Kohlenstoffatomen, vorzugsweise C₈-C₁₀-Phthalat, C₈-C₁₀-Terephthalat, C₈-C₁₀-Isophthalat und C₈-C₁₀-Trimellitat, besonders bevorzugt Di-2-ethylhexylphthalat, Di-isononylphthalat, Di-2-ethylhexylterephthalat, Di-isononylterephthalat, Di-2-ethylhexylisophthalat, Tri-isononylisophthalat, Di-2-ethylhexyltrimellitat und Tri-isononyltrimellitat, bereitgestellt werden.

12. Vorrichtung zum Durchführen eines Verfahrens gemäß einem der Ansprüche 1 bis 11, umfassend eine erste Hydriereinheit (1) und eine oder mehrere weitere Hydriereinheiten (10) und einen oder mehrere Wärmetauscher (7), wobei der Wärmetauscher (7) so angeordnet ist, dass der Ausgangsstrom (3) über einen Eingangsstroms (5) in den Wärmetauscher (7) geleitet wird und der Ausgangsstrom des Wärmetauschers (6a) eine geringere Temperatur als der Eingangsstrom (5) aufweist und anschließend über Rohrleitungen (6b, 6c) als Eingangsstrom (9) in eine weitere Hydriereinheit (10) zugeführt wird und/oder über den Strom (10) in wiederum eine oder mehrere weitere Hydriereinheiten (11) zugeführt wird und/oder über den Strom 8 dem Eingangsstrom zu der ersten Hydriereinheit (1) zugeführt wird, vorzugsweise wobei die Ströme 3 und 9 eine unterschiedliche Temperatur besitzen.

13. Vorrichtung gemäß Anspruch 12, wobei die erste und/oder eine der weiteren Hydriereinheiten (1,11) einen oder mehrere Festbettkatalysatoren aufweist, vorzugsweise wobei der Feststoff-Katalysator mindestens ein Metall der achten Nebengruppe des Periodensystems der Elemente, besonders bevorzugt Ruthenium, aufweist.

14. Vorrichtung gemäß Anspruch 12 oder 13, wobei die Rohrleitungen der ersten Hydriereinheit (1) so angeordnet sind, dass der Ausgangsstrom (3) der ersten Hydriereinheit (1) über einen Bypass (4) oder über die Rohrleitungen (6b, 8) als Eingangsstrom (2b) zu der ersten Hydriereinheit (1) zugeleitet werden kann.

15. Vorrichtung gemäß einem der Ansprüche 11 bis 14, wobei in der ersten und/oder in einer der weiteren Hydriereinheiten eine Mischung aus aromatischen Verbindungen und entsprechenden alizyklischen Verbindungen, vorzugsweise eine Mischung aus aromatische Carbonsäureester und deren entsprechenden alizyklischen Verbindungen mit einer Alkoholkomponente ausgewählt aus der Gruppe bestehend aus verzweigten oder unverzweigten Alkoxyalkyl-, Cycloalkyl- und/oder Alkylgruppen mit 1 bis 25 Kohlenstoffatomen, vorzugsweise ausgewählt aus C₈-C₁₀-Phthalat, C₈-C₁₀-Terephthalat, C₈-C₁₀-Isophthalat und C₈-C₁₀-Trimellitat, besonders bevorzugt Di-2-ethylhexylphthalat, Di-isononylphthalat, Di-2-ethylhexylterephthalat, Di-isononylterephthalat, Di-2-ethylhexylisophthalat, Tri-isononylisophthalat, Di-2-ethylhexyltrimellitat und Tri-isononyltrimellitat, Diisononylphthalat und/oder Didecylphthalat und Di-isononyl-cyclohexandicarbonsäureester und/oder Di-decyl-cyclohexandicarbonsäureester und deren entsprechender alizyklischen Verbindungen, vorhanden ist.
